# EUROPEAN PATENT SPECIFICATION

(11) **EP 1 492 758 B1**
(45) Date of publication and mention of the grant of the patent: **02.06.2010**
(21) Application number: 03712630.7
(22) Date of filing: 24.03.2003
(51) Int. Cl.: C07C 65/40, A61K 31/192

(54) **A COMPOUND AS CHOLINESTERASE INHIBITOR AND ITS ISOLATION FROM FUNGUS SPOROTRICHUM SPECIES**
CHOLINESTERASE-INHIBITOR SOWIE DESSEN ISOLIERUNG AUS DEM PILZ SPOROTRICHUM SPECIES
COMPOSE PRIS COMME INHIBITEUR DE LA CHOLINESTERASE ET PROCEDE ISOLEMENT DE CE COMPOSE A PARTIR DE L ESPECE DE CHAMPIGNON SPOROTRICHUM

(30) Priority: 28.03.2002 US 107806
(43) Date of publication of application: 05.01.2005
(73) Proprietor: Council of Scientific and Industrial Research, New Delhi 110 001 (IN)
(72) Inventor: SHIVANANDAPPA, Thimmappa, Mysore, Karnataka 570 013 (IN); SATTUR, Avinash Prahalad, Mysore, Karnataka 570 013 (IN); SHEREEN, Mysore, Karnataka 570 013 (IN); DIVAKAR, Soundar, Mysore, Karnataka 570 013 (IN); KARANTH, Nayalana Katte Ganesh, Mysore, Karnataka 570 013 (IN)
(74) Representative: Baldock, Sharon Claire
(86) International application number: PCT/IN2003/000073
(87) International publication number: WO 2003/082794

(56) References cited:
- OMURA, SATOSHI ET AL: "Arisugacin, a novel land selective inhibitor of acetylcholinesterase from Penicillium sp. FO-4259" JOURNAL OF ANTIBIOTICS , vol. 48, no. 7, 1995, pages 745-746, XP009013727 cited in the application

## Description

### TECHNICAL FIELD

The present invention relates to a compound 12- (2'-CARBOY- 5'-METHOXYPHENYL)-2,12-DIHYDROXY-DODECA-4-ONE "Sporotricolone", mainly as acetylcholinesterase (AchE) inhibitor. The present invention also relates to a process for the isolation of said compound from fungus *Sporotrichum* species.

### BACKGROUND ART

Enzyme inhibitors are important class of molecules that are used as drugs and pesticides. The enzyme acetylcholinesterase (AchE) is involved in the synaptic transmission of the nerve impulse and its inhibition leads to accumulation of the neurotransmitter, acetylcholine leading to overexcitation of the postsynaptic neuron. This property of the inhibitor has been exploited to develop newer insecticides against a wide range of insect pests as well as drugs effective against worms, and, recently a new class of neuroactive drugs against dementia (Alzheimer's).

Although earlier authors have isolated metabolites such asasteric acis, questin and questinol from *Sorortricum* sp., no AchE inhibitor activity has been reported (Slater, GP, Haskins, RH and Hogge, L.R. Can J Mirobiol 17 (1971), 1576-79). The fungi, *Aspergillus terreus* (Ling, KH, Liou,HH, Yang, CM and Yang CK, Appl. Env. Microbiol, 37 (1979) 355-57) and *Penicillium* sp. (Omura, Skuno,F, Otoguro,K, Shiomi,K. Mauma,R and Iwai.Y. J. Antibiot. 48(1995) 745-46) have been reported to produce an AchE inhibitor named Arusigacin. However, the AchE inhibitor of the present invention is isolated from Sporotrichum having distinct chemical structure and properties and therefore a novel inhibitor molecule.

After screening of various microorganisms, a fungal culture is selected which shows inhibition against a serine esterase/ protease/cholinesterase enzyme. This imperfect deuteromycetes, *Sporotrichum* species and was first isolated in 1966. The taxonomic features of *Sporotrichum* species (deuteromyces) are broad hyphae and septate in nature; has hyalline conidiophores with little differentiation from vegetatative hyphae and solitary conidia with broad attachment to the hyphae.

This culture has previously been a subject of research investigation at the Central Food Technological Research Institute (CFTRI) India, for its ability to grow on lignocellulosic wastes for the production of enzymes and organic acids (Sreekantaiah, KR, PhD thesis (1976) University of Mysore; Manonmani, HK, PhD thesis (1986) University of Mysore). This culture has now been used in the present invention to produce a fermented extract containing a serine esterase / protease/ cholinesterase inhibitor.

The conditions of fermentation have been described earlier in Indian Patent Application No. 303/DEL/2000 Sattur, AP, Shivanandappa, T, Divakar, S and Karanth, NG.

### OBJECTS OF THE INVENTION

The main object of the present invention is to provide bioactive compound 12- (2'-CARBOXY-5'-METHOXYPHENYL)-2,12-DIHYDROXY-DODECA-4-ONE "Sporotricolone" having inhibitory activity against acetylcholinesterase (AchE).

Another object of the present invention is to provide a compound having inhibitory activity against seiren esterase and protease.

Yet another object of the present invention is to provide a compound having insecticidal properties.

Still another object of the present invention is to provide a compound having enhanced cholinergic activity.

Yet another object of the present invention is to provide a process for the isolation of the above said compound.

### SUMMARY OF THE INVENTION

The present invention provides a compound 12- (2'-CARBOXY- 5'-METHOXYPHENYL)-2,12-DIHYDROXY-DODECA-4-ONE "Sporotricolone" mainly as acetylcholinesterase (AchE) inhibitor. The present invention also provides a process for the isolation of said compound from fungus *Sporotrichum* species.

### DETAILED DESCRIPTION OF THE INVENTION

Accordingly, the present invention provides a bioactive compound 12- (2'-CARBOXY-5'-METHOXYPHENYL)-2,12-DIHYDROXY-DODECA-4-ONE "Sporotricolone" of formula (I) having acetylcholinesterase (AchE) enzyme inhibition activity obtained from fungus *Sporotrichum* species.

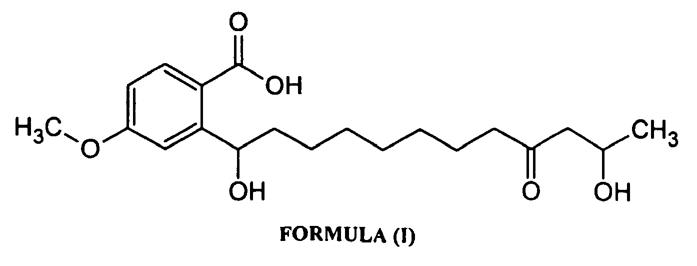

An embodiment of the present invention wherein the compound 12- (2'-CARBOXY- 5'-METHOXYPHENYL)-2,12-DIHYDROXY-DODECA-4-ONE "Sporotricolone" of formula (I) having the following characteristic properties:
**Solubility:** Highly soluble in ethyl acetate, methanol and acetone.
**UV (ethyl acetate) λₘₐₓ:** 265 nm, 312 nm.
**¹HNMR spectrum (DMSO, δ_{TMS}=0.00 ppm);**
δ 1.03 (3H, d, J=6.3Hz, -CH-CH₃)
δ 1.2-2.7 (14H, m, 7x - CH₂)
δ 3.6-3.8 (m, Ar-O-CH₃ and Ar-CH-OH)
δ 7.20 (1H, d, J=2.5Hz, C_{6'}-H)
δ 7.30 (2H, d, J = 7.1 Hz, C₃, -H and C_{4'}-H)
**Mass spectrum (EI, 70 eV, 25° C, 200-ul amp):**
m/e: 336 (M+), 279(366-87), 167(274-112), 57 (CH₂COCH₃), 43, 29.

Another embodiment of the present invention, wherein the purity of the compound is established by TLC and RP HPLC.

Yet another embodiment of the present invention, wherein said compound is named as 12-(2'-CARBOXY- 5'-METHOXYPHENYL)-2,12-DIHYDROXY-DODECA-4-ONE "Sporotricolone".

Still another embodiment of the present invention, wherein said compound is an inhibitor of the enzyme acetylcholinesterase from the rat brain as well as erythrocytes with a IC₅₀ value of 20 x 10⁻⁶ M.

Yet another embodiment of the present invention, wherein said compound also acts as an inhibitor of serine esterase of the rat liver serum.

Still another embodiment of the present invention, wherein said compound having insecticidal properties.

Yet another embodiment of the present invention, wherein said compound effective against mosquito larvae at an optimum concentration of 70 µg/ml water (70 ppm) when exposed for 24 hrs.

Still another embodiment of the present invention, wherein the insecticidal activity of the compound against mosquito larvae is selected from *culex quinquifasciatus.*

Yet another embodiment of the present invention, wherein said compound as acetylcholineesterase inhibitor having potential application as a drug for Alzheimer's disease or dermentia.

The present invention also provides a process for the isolation of 12- (2'-CARBOXY- 5'-METHOXYPHENYL)-2,12-DIHYDROXY-DODECA-4-ONE Sporotricolone from the fungus Sporotrichum species, said process comprising the steps of:
(a) extracting the fermented solid with an organic solvent;
(b) filtering the extract of step (a) through a cloth or Whatman filter paper to obtain a clear solution;
(c) evaporating the solution of step (b) under reduced pressure to obtain a crude extract;
(d) purifying the crude extract of step (c) by column chromatography over silica gel and eluting with mixture of organic solvents of increasing polarity;
(e) pooling active eluted fraction of step (d) and further subjected to column chromatography over silica gel by eluting with mixture of organic solvents with increasing polarity;
(f) repooling the active eluted fractions of step (e);
(g) evaporating the pooled fractions of step (f) to get a residue; and
(h) dissolving the residue in step (g) in ethyl acetate to yield the pure compound "Sporotricolone".

Yet another embodiment of the present invention, a process wherein in step (a) the organic solvent is selected from a group consisting of ethyl acetate, acetone or methanol and preferably ethyl acetate.

Still another embodiment of the present invention, a process wherein in step (d) the mixture of organic solvents is selected from the combination of hexane: diethyl ether and chloroform: methanol mixtures.

Further embodiment of the present invention, a process wherein in step (e) the mixture of organic solvent used is chloroform: ethyl acetate mixture.

Yet another embodiment of the present invention, wherein the said compound is separated and purified by column chromatography on silica gel and RP HPLC.

Still another embodiment of the present invention, wherein said compound having an UV absorption at 265 and 312 nm.

The present invention is further explained in the form of following embodiments

In the present invention a process for the isolation of an acetylcholinesterase inhibitor, which comprises the extraction of the fermented broth culture with solvents such as ethyl acetate. The crude extract is further extracted with 10-20 ml of methanol and subjected to column chromatography using silica gel and eluted with various combinations of solvents such as hexane: diethyl ether (85:15, 50:50) followed by chloroform: methanol (95:5, 50:50, 10:90). Fractions are evaporated under nitrogen, dissolved in ethyl acetate and assayed for acetylcholinesterase (AchE) inhibition. The active fractions are pooled and further subjected to purification on silica gel column chromatography and eluted with chloroform: ethyl acetate (90:10, 50:50, 0:100). The active fractions pooled and the solvent evaporated and dissolved in 2 ml ethyl acetate. The purity, as checked by TLC, showed a single spot and HPLC on reverse phase column (C 18) with chloroform and methanol as mobile phase. The yield is about 10 mg. The purified inhibitor showed inhibitor potency against rat brain AchE with an IC₅₀ of 15-20 x 10⁻⁶ M.

A general process for the production of the novel Acetylcholinesterase inhibitor is given in the flow sheet:

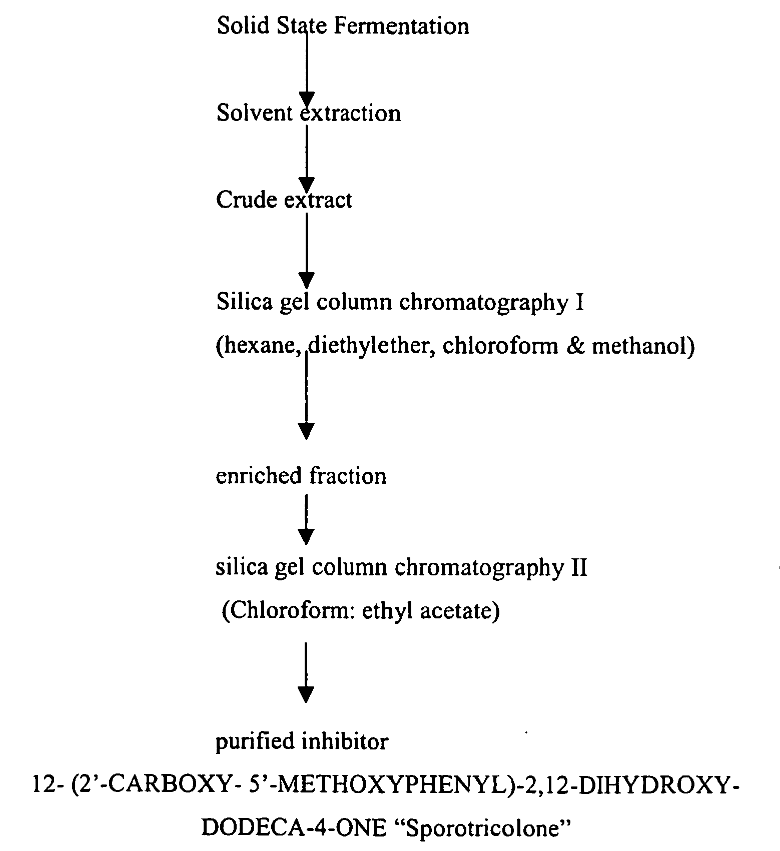

The structure of the isolated inhibitor is determined by UV, ¹H NMR and mass spectrometry. The following examples are given by way of illustration of the present invention and should not be construed to limit the scope of the invention.

### EXAMPLE - 1

The fermentation culture is extracted with 100 ml of ethyl acetate, filtered through a cotton filter and concentrated in vacuo to obtain 1 ml of the crude extract. This is used as a source of the enzyme inhibitor and 20 µl of the extract gave 60-90 % inhibition of rat brain acetylcholinesterase enzyme.

### EXAMPLE -2

The crude extract is further extracted with 10-20 ml of methanol and subjected to column chromatography using silica gel and eluted with various combinations of solvents such as hexane: diethyl ether (85:15, 50:50) followed by chloroform: methanol (95:5, 50:50, 10:90). Fractions are evaporated under nitrogen, dissolved in ethyl acetate and assayed for acetyl cholinesterase (AchE) inhibitor. The active fractions (# 11-19) are pooled and further subjected to purification on silica gel column chromatography and eluted with chloroform: ethyl acetate (90:10, 50:50, 0:100). The active fractions pooled and the solvent evaporated and dissolved in 2 ml ethyl acetate. The purity, as checked by TLC, showed a single spot. RP HPLC also ascertained the purity on a C18 column with chloroform and methanol as the mobile phase wherein it is a single peak. The yield is about 10 mg.

### EXAMPLE - 3

The purified inhibitor showed inhibitor potency against rat brain AchE with an IC50 of 15-20 x 10⁻⁶ M. as assayed according to Ellman et al., (Biochem. Pharmacol. 7(1961), 88-95) and is given as follows: The enzyme inhibition is carried out by pre- incubating the enzyme (rat brain acetylcholinesterase) with 2-20ul of the culture extract or the column fraction at room temperature (25 °C) for 15 minutes followed by the addition of the substrate, acetyl thiocholine iodide (0.5mM), in 3ml phosphate buffer (0.1.M, pH.7.4) containing 0.25mM dithiobisnitrobenzoic acid. Absorbance change at 412 nm is monitored every 30 seconds for 2 min in an UV-VIS Spectrophotometer. Inhibition is calculated relative to the solvent control. IC₅₀ is determined by regression analysis.

### EXAMPLE - 4

Inhibition of serine esterase: The isolated compound (inhibitor) was demonstrated to be an inhibitor of serine esterase as follows:
Inhibition of esterase activity was assayed *in vitro* with the substrate, *p*-nitrophenyl acetate in tris buffer (.05 M, pH 7.4) and the rat liver extract as the source of the enzyme. The inhibitor, at various concentrations, was pre-incubated with the enzyme in buffer at room temperature for 30 minutes and the residual activity was measured. The product of enzymatic hydrolysis (*p*-nitrophenol) was monitored spectrophotometrically by measuring the absorbance change per minute at 405 nm, according to the method of Sidhu and Blair (J. Biol. Chem., 1975, 250: 7891). Percentage inhibition was calculated with respect to the control (without inhibitor) and IC₅₀ (the concentration required to inhibit 50% activity) was calculated by regression analysis. IC₅₀ for the microbial inhibitor was found to be 0.067 µmole/ml which indicates that it is a potent inhibitor of serine esterase.

### EXAMPLE - 5

Insecticidal properties: The insecticidal property of the inhibitor isolated from Sporotrichum sps was investigated as follows:

### a. Toxicity to mosquito larvae:

Late III instar larvae of the mosquito, *Culex quinquefaciatus* (from laboratory culture) were released in four beakers containing tap water (ten larvae in each beaker). The treatment group (2 replicates) was added the inhibitor compound at 100 ppm in 100 µl acetone whereas the control group received acetone only. After 24 hrs, 75 % mortality (7/10 and 8/10) was observed in treated group whereas no mortality was seen in the control group (0/10, 0/10). This experiment demonstrates the insecticidal activity of the compound.

### b. Acetyl cholinesterase of the housefly brain:

Insecticidal potential of the compound was further investigated by the inhibition of the target enzyme, acetylcholinesterase(AchE) from the housefly brain *in vitro.* Ten house fly heads were homogenized in 3 ml buffer(tris .05M, pH 7.4) which served as the source of the enzyme. AchE inhibition was assayed with acetyl thiocholine as the substrate with various concentrations of the inhibitor as described earlier for the rat brain enzyme. Percentage inhibition was calculated with respect to the control (without inhibitor) and IC₅₀ determined by regression analysis. The compound was found to be a potent inhibitor of the housefly brain AchE with an IC₅₀ value of 0.98 µmole/ml.

From the above experiment, it is very clear that the compound of the invention is effective in killing or inhibiting the growth of insects, and evidence that the compound functions as an acetylcholinesterase inhibitor. It is well known to those of skill in the art that acetylcholinesterase inhibitors are effective as insecticides.

### MAIN ADVANTAGES OF THE PRESENT INVENTION

1. The present invention provides an AchE /serine esterase/protease inhibitor from a microbial source.
2. The present invention provides a simple extraction and chromatographic procedure to purify the AchE inhibitor from the crude mixture.
3. In the present invention the isolated inhibitor is a novel bioactive molecule

## Claims

1. A compound having the formula:

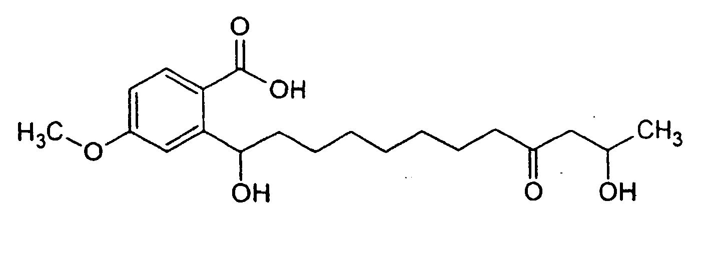

2. Use of the compound of formula 1 for killing or inhibiting the growth of an insect comprising administering to the insect an effective amount of the compound.

3. Use as claimed in claim 2 wherein said insect is a mosquito.

4. Use as claimed in claim 3 wherein said mosquito is *Culex quinquifasciatus.*

5. The compound according to claim 1 is as an inhibitor of serine esterase.

6. A compound as defined in claim 1 for application as a drug for Alzheimer's disease or dementia.

7. A process for the isolation of the compound according to claim 1, from the fungus Sporotrichum species, said process comprising the steps of:
(a) extracting fermented solid with an organic solvent;
(b) filtering the extract of step (a) through a cloth or Whatman filter paper to obtain a clear solution;
(c) evaporating the solution of step (b) under reduced pressure to obtain a crude extract;
(d) purifying the crude extract of step (c) by column chromatography over silica gel and eluting with mixture of organic solvents of increasing polarity;
(e) pooling active eluted fractions of step (d) and further subjected to column chromatography over silica gel by eluting with a mixture of organic solvents with increasing polarity;
(f) repooling the active eluted fractions of step (e);
(g) evaporating the pooled fractions of step (f) to yield the pure compound of formula I and
(h) dissolving the residue in step (g) in ethyl acetate to yield the pure compound of claim 1.

8. The process according to claim 7, wherein in step (a) the organic solvent is selected from the group consisting of ethyl acetate, acetone and methanol.

9. The process according to claim 7, wherein in step (d) the mixture of organic solvents is selected from the combination of hexane: diethyl ether and chloroform: methanol mixtures.

10. The process according to claim 7, wherein in step (e) the mixture of organic solvent used is chloroform: ethyl acetate mixture.

## Patentansprüche

1. Verbindung der Formel:

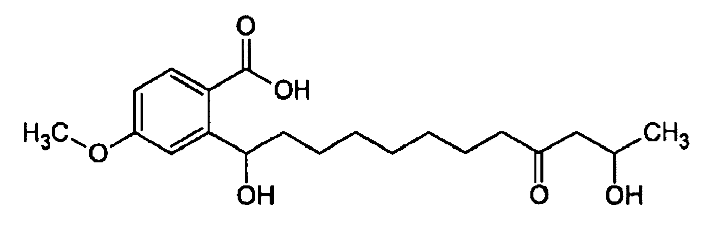

2. Verwendung der Verbindung der Formel 1, um ein Insekt zu töten oder das Wachstum eines Insekts zu hemmen, die die Anwendung einer wirksamen Menge der Verbindung bei dem Insekt umfasst.

3. Verwendung nach Anspruch 2, wobei das Insekt eine Stechmücke ist.

4. Verwendung nach Anspruch 3, wobei es sich bei der Stechmücke um Culex quinquifasciatus handelt.

5. Verbindung nach Anspruch 1 als Inhibitor der Serin-Esterase.

6. Verbindung, wie sie in Anspruch 1 definiert ist, für die Anwendung als Arzneimittel bei Alzheimer-Krankheit oder Demenz.

7. Verfahren zur Isolierung der Verbindung nach Anspruch 1 aus Sporotrichum Arten, wobei das Verfahren die folgenden Schritte umfasst:
(a) fermentierter Feststoff wird mit einem organischen Lösemittel extrahiert;
(b) der Extrakt aus Schritt (a) wird zur Bildung einer klaren Lösung an einem Tuch oder an Whatman-Filterpapier filtriert;
(c) die Lösung aus Schritt (b) wird zur Bildung eines Rohextraktes unter vermindertem Druck eingedampft;
(d) der Rohextrakt aus Schritt (c) wird säulenchromatographisch an Kieselgel gereinigt und mit einem Gemisch von Lösemitteln steigender Polarität eluiert;
(e) die eluierten Fraktionen aus Schritt (d) werden vereinigt und weiter säulenchromatographisch an Kieselgel behandelt, wobei mit einem Gemisch von Lösemitteln steigender Polarität eluiert wird;
(f) die eluierten Fraktionen aus Schritt (e) werden wieder vereinigt;
(g) die vereinigten Fraktionen aus Schritt (f) werden eingedampft, wodurch die reine Verbindung der Formel 1 anfällt; und
(h) der Rückstand aus Schritt (g) wird in Ethylacetat gelöst, wodurch die reine Verbindung des Anspruchs 1 anfällt.

8. Verfahren nach Anspruch 7, wobei in Schritt (a) das organische Lösemittel unter Ethylacetat, Aceton und Methanol ausgewählt ist.

9. Verfahren nach Anspruch 7, wobei in Schritt (d) das Gemisch von organischen Lösemitteln unter den Kombinationen Hexan:
Diethylether-Gemisch und Chloroform: Methanol-Gemisch ausgewählt ist.

10. Verfahren nach Anspruch 7, wobei in Schritt (e) das verwendete Gemisch von organischen Lösemitteln das Chloroform: Ethylacetat-Gemisch ist.

## Revendications

1. Composé ayant la formule:

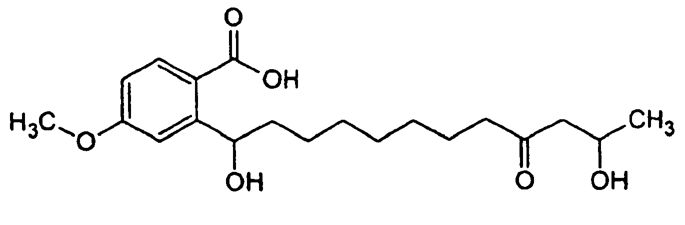

2. Utilisation du composé de la formule 1 pour sacrifier ou inhiber la croissance d'un insecte comprenant l'administration à l'insecte d'une quantité efficace du composé.

3. Utilisation selon la revendication 2, où ledit insecte est un moustique.

4. Utilisation selon la revendication 3, dans laquelle ledit moustique est *Culex quinquifasciatus.*

5. Composé selon la revendication 1 en tant qu'inhibiteur de l'estérase de sérine.

6. Composé selon la revendication 1, pour l'application comme un médicament pour la maladie d'Alzheimer ou la démence.

7. Processus d'isolation du composé selon la revendication 1, de l'espèce fungus Sporotrichum, ledit processus comprenant les étapes de:
(a) extraire un solide fermenté avec un solvant organique;
(b) filtrer l'extrait de l'étape (a) à travers un tissu ou un papier filtrant de Whatman pour obtenir une solution claire;
(c) faire évaporer la solution de l'étape (b) sous pression réduite pour obtenir un extrait brut;
(d) purifier l'extrait brut de l'étape (c) par chromatographie sur colonne sur gel de silice et éluer avec le mélange de solvants organiques d'une polarité en augmentation;
(e) mélanger des fractions éluées actives de l'étape (d) et les soumettre en outre à une chromatographie sur colonne sur du gel de silice en éluant avec un mélange de solvants organiques d'une polarité en augmentation;
(f) remélanger les fractions éluées actives de l'étape (e) ;
(g) faire évaporer les fractions mélangées de l'étape (f) pour obtenir le composé pur de la formule 1 et
(h) dissoudre le résidu à l'étape (g) dans de l'éthyl acétate pour obtenir le composé pur de la revendication 1.

8. Processus selon la revendication 7, dans lequel dans l'étape (a), le solvant organique est sélectionné dans le groupe consistant en éthyl acétate, acétone et méthanol.

9. Processus selon la revendication 7, dans lequel dans l'étape (d), le mélange de solvants organiques est sélectionné dans la combinaison de mélanges hexane: diéthyl éther et chloroforme: méthanol.

10. Processus selon la revendication 7, dans lequel dans l'étape (e), le mélange de solvants organiques utilisé est le mélange de chloroforme: éthyl acétate.
